# EUROPEAN PATENT APPLICATION

(11) **EP 4 053 465 A2**
(43) Date of publication of application: **07.09.2022**
(21) Application number: 22155845.5
(22) Date of filing: 09.02.2022
(51) Int. Cl.: F24F 8/108, F24F 11/62, F24F 11/70, G01N 27/00, G01N 33/00, F24F 11/54

(54) **METHOD FOR DETECTING AND FILTERING INDOOR POLLUTED GAS**

(30) Priority: 03.03.2021 TW 110107602
(71) Applicant: Microjet Technology Co., Ltd., Hsinchu (TW)
(72) Inventor: Mou, Hao-Jan, Hsinchu (TW); Wu, Chin-Chuan, Hsinchu (TW); Han, Yung-Lung, Hsinchu (TW); Huang, Chi-Feng, Hsinchu (TW); Tsai, Chang-Yen, Hsinchu (TW); Lee, Wei-Ming, Hsinchu (TW); Kuo, Chun-Yi, Hsinchu (TW)
(74) Representative: Uexküll & Stolberg

(57) **Abstract**

A method for detecting and filtering indoor polluted gas includes providing a plurality of gas detection devices (1a, 1b, 1c 1d) to detect the polluted gas in an indoor space; providing a plurality of filtration and purification devices (3a, 3b, 3c, 3d) to filter the polluted gas and to receive a first control command through a wireless communication to enable filtration of the polluted gas; and providing a connection device (2) to perform intelligent computation to receive and compare the data of the polluted gas detected by the gas detection devices (1a, 1b, 1c 1d) to perform intelligent computation to figure out a location of the polluted gas and intelligently and selectively transmit the first control command to a filtration and purification device (3a, 3b, 3c, 3d) which at the location of the polluted gas, thereby filtering the polluted gas and retaining the polluted gas from spreading outwardly, allowing the polluted gas in the indoor space to become a clean, safe, and breathable gas.

## Description

### FIELD OF THE INVENTION

The present disclosure relates to a gas filtering method implemented in an indoor space, so that the polluted gas in the indoor space can be quickly filtered to be a clean, safe, and breathable gas.

### BACKGROUND OF THE INVENTION

In the light of people pay more and more attention to the ambient air quality in daily life by nowadays, it is understood that gases containing particulate matters (PM1, PM2.5, PM10), carbon dioxide, total volatile organic compounds (TVOC), formaldehyde, etc. or even the particulates, the aerogels, the bacteria, the viruses in the gas might result in adverse effects on the human health, even might be life-threatening when exposure to these gases.

As stated above, it is not easy to control the indoor gas quality since the affecting factors include not only the outdoor space gas quality but also the air conditioning and the pollution source in the indoor space (especially the dusts originated from poor circulation of air in the indoor space). In order to improve the indoor gas quality, air conditioners or air cleaners can be utilized. However, air conditioning device such as the air conditioner and the air cleaner lack the function of real time monitoring indoor gas quality for engaging the air filtration instantly, but the user has to enable the air filtration of the air conditioning device passively. As a result, the issue of the gas quality of the gas in the indoor space cannot be solved completely.

Consequently, it is an issue of the present invention to provide a solution that can instantly purify and improve the gas quality in the indoor space, reducing the risks of inhaling the hazardous gases and monitoring the gas quality in the indoor space anytime and anywhere.

### SUMMARY OF THE INVENTION

One object of the present disclosure is to provide a method for detecting and filtering indoor polluted gas. In the method, according to one or some embodiments of the present disclosure, a connection device is provided to receive and compare the data of the polluted gas detected by a plurality of gas detection devices to perform intelligent computation for finding out the location in the indoor space containing the polluted gas. The connection device intelligently and selectively transmits a control command to enable a filtration and purification device at the location of the indoor space containing the polluted gas for filtering the polluted gas, and allowing the polluted gas in the indoor space to become a clean, safe, and breathable gas.

In view of the above object, a method for detecting and filtering indoor polluted gas is provided. The method includes: providing a plurality of gas detection devices to detect the polluted gas in the indoor space; providing a plurality of filtration and purification devices to filter the polluted gas and receive a first control command through a wireless communication to enable filtration of the polluted gas; providing a connection device to perform intelligent computation to receive and compare the data of the polluted gas detected by the gas detection devices to perform intelligent computation for finding out a location in the indoor space containing the polluted gas and intelligently and selectively transmit the first control command to a filtration and purification device which at the location of the indoor space containing the polluted gas, thereby enabling the filtration and purification device to filter and avoid the spreading outwardly of the polluted gas, allowing the polluted gas in the indoor space to become a clean, safe, and breathable gas.

### BRIEF DESCRIPTION OF THE DRAWINGS

The disclosure will become more fully understood from the detailed description given herein below, for illustration only and thus not limitative of the disclosure, wherein:
FIG. 1 illustrates a schematic view (1) for the indoor space operation of a method for detecting and filtering indoor polluted gas of an exemplary embodiment of the present disclosure;
FIG. 2 illustrates a schematic view (2) for the indoor space operation of the method for detecting and filtering indoor polluted gas of the exemplary embodiment of the present disclosure;
FIG. 3 illustrates a cross-sectional view of a filtration and purification device of the exemplary embodiment of the present disclosure;
FIG. 4 illustrates a schematic perspective view of a gas detection device of the exemplary embodiment of the present disclosure;
FIG. 5A illustrates a schematic perspective view (1) of a gas detection main body of the exemplary embodiment of the present disclosure;
FIG. 5B illustrates a schematic perspective view (2) of the gas detection main body of the exemplary embodiment of the present disclosure;
FIG. 5C illustrates an exploded view of the gas detection main body of the exemplary embodiment of the present disclosure;
FIG. 6A illustrates a perspective view (1) of a base of the exemplary embodiment of the present disclosure;
FIG. 6B illustrates a perspective view (2) of the base of the exemplary embodiment of the present disclosure;
FIG. 7 illustrates a perspective view (3) of the base of the exemplary embodiment of the present disclosure;
FIG. 8A illustrates an exploded view showing that a piezoelectric actuator is to be disposed in the base, according to the exemplary embodiment of the present disclosure;
FIG. 8B illustrates a perspective view showing that the piezoelectric actuator is disposed in the base, according to the exemplary embodiment of the present disclosure;
FIG. 9A illustrates an exploded view (1) of the piezoelectric actuator of the exemplary embodiment of the present disclosure;
FIG. 9B illustrates an exploded view (2) of the piezoelectric actuator of the exemplary embodiment of the present disclosure;
FIG. 10A illustrates a cross-sectional view (1) showing the operation of the piezoelectric actuator of the exemplary embodiment of the present disclosure;
FIG. 10B illustrates a cross-sectional view (2) showing the operation of the piezoelectric actuator of the exemplary embodiment of the present disclosure;
FIG. 10C illustrates a cross-sectional view (3) showing the operation of the piezoelectric actuator of the exemplary embodiment of the present disclosure;
FIG. 11A illustrates a cross-sectional view (1) showing the operation of the gas detection module of the exemplary embodiment of the present disclosure;
FIG. 11B illustrates a cross-sectional view (2) showing the operation of the gas detection module of the exemplary embodiment of the present disclosure; and
FIG. 11C illustrates a cross-sectional view (3) showing the operation of the gas detection module of the exemplary embodiment of the present disclosure.

### DETAILED DESCRIPTION OF THE PREFERRED EMBODIMENT

The present disclosure will now be described more specifically with reference to the following embodiments. It is to be noted that the following descriptions of different embodiments of this disclosure are presented herein for the purpose of illustration and description only, and it is not intended to limit the scope of the present disclosure.

According to one or some embodiments of the present disclosure, a method for detecting and filtering indoor polluted gas is provided and is adapted to figure out a polluted gas in an indoor space for filtration. The method includes providing a plurality of gas detection devices to detect the polluted gas in the indoor space, providing a plurality of filtration and purification devices to filter the polluted gas and receive a first control command through a wireless communication to enable filtration of the polluted gas; providing a connection device to perform intelligent computation to receive and compare the data of the polluted gas detected by the gas detection devices to perform intelligent computation for finding out a location in the indoor space containing the polluted gas and intelligently and selectively transmit the first control command to a filtration and purification device which at the location of the indoor space containing the polluted gas, thereby filtering the polluted gas and retaining the polluted gas from spreading outwardly, thus allowing the polluted gas in the indoor space to become a clean, safe, and breathable gas.

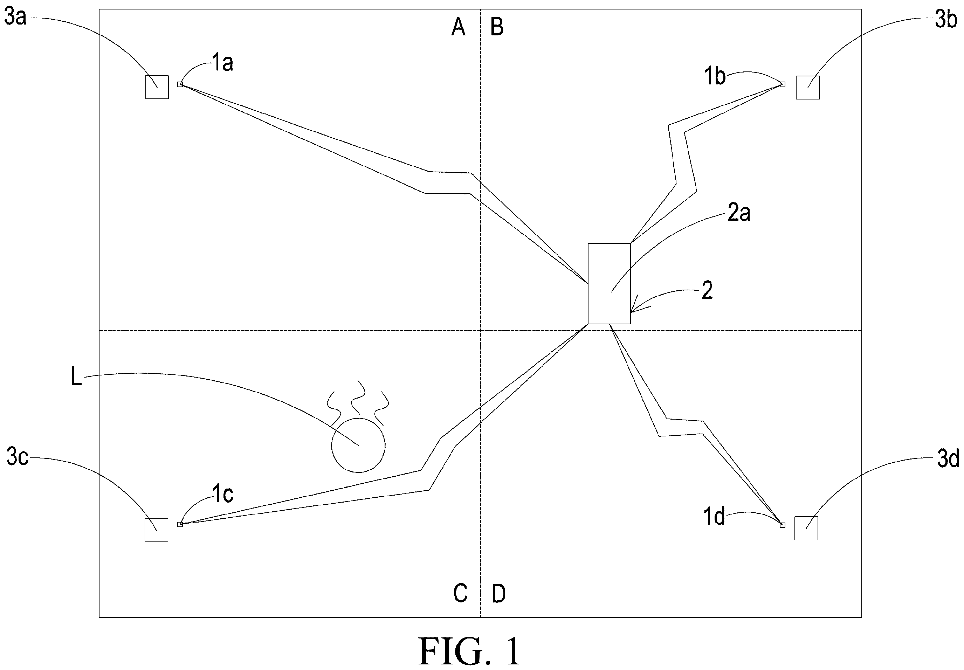

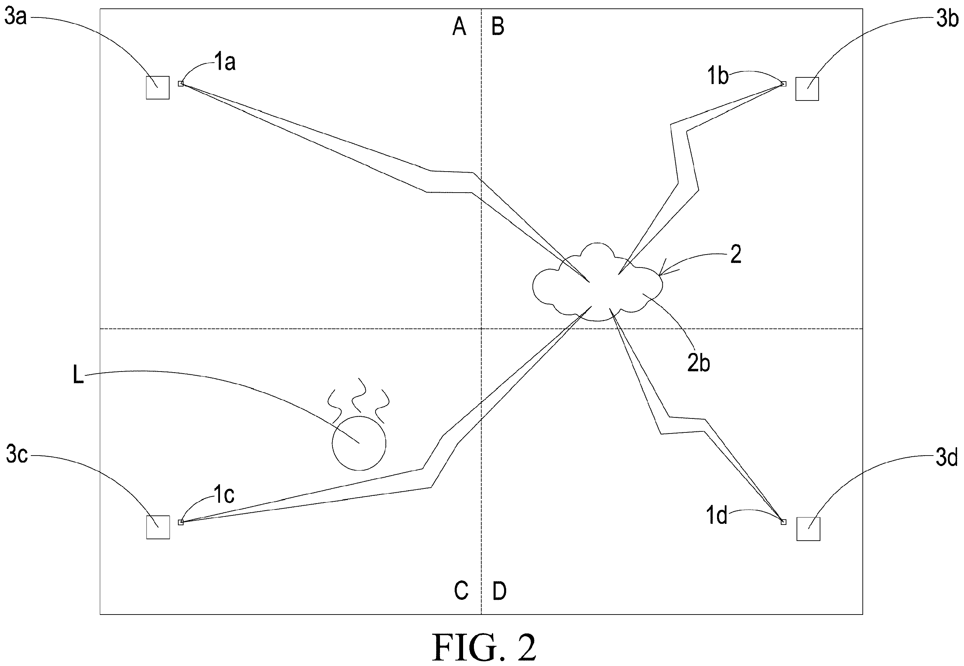

Please refer to FIG. 1 and FIG. 2, an exemplary embodiment of the present disclosure is described as below. According to the method of one or some embodiments of the present disclosure, a plurality of gas detection devices la, 1b, 1c, 1d, a connection device 2, and a plurality of filtration and purification devices 3a, 3b, 3c, 3d are disposed in an indoor space. For clarity, the area at which the gas detection device la, the filtration and purification device 3a are located is defined as the area A; the area at which the gas detection device 1b, the filtration and purification device 3b are located is defined as the area B; the area at which the gas detection device 1c, the filtration and purification device 3c are located is defined as the area C; also the area at which the gas detection device 1d, the filtration and the purification device 3d are located is defined as the area D. Each of the gas detection devices la, 1b, 1c, 1d is provided for detecting a data of the polluted gas in the corresponding area A, B, C, D to output the data to the connection device 2.

The connection device 2 receives and compares the data of the polluted gas by the gas detection devices la, 1b, 1c, 1d to perform intelligent computation for finding out whether the location L of the indoor space containing the polluted gas is located in the area A, the area B, the area C, or the area D. The connection device 2 then intelligently and selectively transmits a first control command to the filtration and purification device 3a, 3b, 3c, 3d at the location L through a wired communication or a wireless communication to enable the filtration and purification device 3a, 3b, 3c, 3d at the area L to filter the polluted gas.

Please refer to FIG. 1 and Table 1 below. Table 1 shows the data of the polluted gas in the location L detected by the gas detection devices la, 1b, 1c, 1d corresponding to the area A, B, C, D of the indoor space. According to one embodiment of the present invention, the location L which contains the polluted gas in the area C is determined after the gas detection devices la, 1b, 1c, 1d transmit the data of the polluted gas to the connection device 2 for performing intelligent computation, thereby the closest to the location L containing the polluted gas is the filtration and purification device 3c in the area C. Next, the connection device 2 transmits the first control command to the filtration and purification device 3c, enabling the filtration and purification device 3c to filter and purify the polluted gas, also the connection device 2 performs intelligent computation to transmit a second control command to rest of the filtration and purification devices 3a, 3b, 3d to accelerate the filtration of the polluted gas, allowing the polluted gas in the indoor space to become a clean, safe, and breathable gas.

**Table 1**

| | | | | | | | | | | | | | | |
|---|---|---|---|---|---|---|---|---|---|---|---|---|---|---|
| 2.72 | 3.93 | 5.30 | 6.69 | 7.91 | 8.74 | 9.04 | 8.74 | 7.91 | 6.69 | 5.30 | 3.93 | 2.72 | 1.76 | 1.07 |
| 3.93 | 5.67 | 7.65 | 9.66 | 11.41 | 12.61 | 13.04 | 12.61 | 11.41 | 9.66 | 7.65 | 5.67 | 3.93 | 2.55 | 1.54 |
| 5.30 | 7.65 | 10.32 | 13.04 | 15.40 | 17.02 | 17.60 | 17.02 | 15.40 | 13.04 | 10.32 | 7.65 | 5.30 | 3.44 | 2.08 |
| 6.69 | 9.66 | 13.04 | 16.46 | 19.45 | 21.50 | 22.22 | 21.50 | 19.45 | 16.46 | 13.04 | 9.66 | 6.69 | 4.34 | 2.63 |
| 7.91 8.74 | 11.41 12.61 | 15.40 17.02 | 19.45 21.50 | 22.98 25.39 | 25.39 28.07 | 26.26 29.02 | 25.39 28.07 | 22.98 25.39 | 19.45 21.50 | 15.40 17.02 | 11.41 12.61 | 7.91 8.74 | 5.13 5.67 | 3.11 3.44 |
| 9.04 | 13.04 | 17.60 | 22.22 | 26.26 | 30.00 | 29.05 | 29.02 | 26.26 | 22.22 | 17.60 | 13.04 | 9.04 | 5.86 | 3.55 |
| 8.74 | 12.61 | 17.02 | 21.50 | 25.39 | 28.07 | 29.02 | 28.07 | 25.39 | 21.50 | 17.02 | 12.61 | 8.74 | 5.67 | 3.44 |
| 7.91 | 11.41 | 15.40 | 19.45 | 22.98 | 25.39 | 26.26 | 25.39 | 22.98 | 19.45 | 15.40 | 11.41 | 7.91 | 5.13 | 3.11 |
| 6.69 | 9.66 | 13.04 | 16.46 | 19.45 | 21.50 | 22.22 | 21.50 I | 19.45 | 16.46 | 13.04 | 9.66 | 6.69 | 4.34 | 2.63 |

The method for detecting and filtering indoor polluted gas according to one or some embodiments of the present disclosure is further described. In this embodiment, the connection device 2 performs intelligent computation to figure out the location L of the polluted gas by a trilateration. Firstly, the positions of any three of the gas detection devices (e.g., the gas detection devices la, 1b, 1c) are located. Subsequently, by taking the gas detection devices la, 1b, 1c as the center of circles to determine the distances between the gas detection devices la, 1b, 1c and the polluted gas. Therefore, the location L of the polluted gas can be located. Please refer to the table 1 as an instance, the location L of the polluted gas is in the area C of the indoor space and the value of the data is 30. Therefore, the value of the data detected by the gas detection device 1c closest to the location L of the polluted gas is 6.69 (the highest), the value of the data detected by the gas detection device 1b furthest from the location L of the polluted gas is 1.07, and the values of the data detected by the rest two gas detection devices 1a, 1d are 2.72 and 2.63, respectively. Hence, the positions of the three gas detection devices la, 1b, 1c are taken as the center of circles, and the values of the data detected by the three gas detection devices la, 1b, 1c, namely, 2.72, 1.07, and 6.69, are taken as the basis, and the distances between the location L of the polluted gas and the three gas detection devices la, 1b, 1c are located. When the positions of the three gas detection devices la, 1b, 1c are determined, and the distances between the location L of the polluted gas and the three gas detection devices la, 1b, 1c are calculated, then the location L of the polluted gas can be located by trilateration. It should be noted that, in this embodiment, the trilateration is applied, but embodiments are not limited thereto; in some embodiments, more than three points can be applied in the fix method to estimate the location L of the polluted gas.

In this embodiment, each of the gas detection devices 1a, 1b, 1c, 1d may stationary or movable. Moreover, the connection device 2 may be a mobile device 2a or a cloud processing device 2b.

According to one or some embodiments, when the connection device 2 performs intelligent computation to locate the location L of the polluted gas is in the area C of the indoor space, the connection device 2 transmits the first control command to the filtration and purification device 3c which is closest to the location L to enable the filtration and purification device 3c for filtering and purifying the polluted gas, and subsequently the connection device 2 transmits the second control command to the rest three filtration and purification devices 3a, 3b, 3d for enabling the filtration and purification devices 3a, 3b, 3d to filter and purify to accelerate the filtration of the polluted gas, allowing the polluted gas in the indoor space to become a clean, safe, and breathable gas.

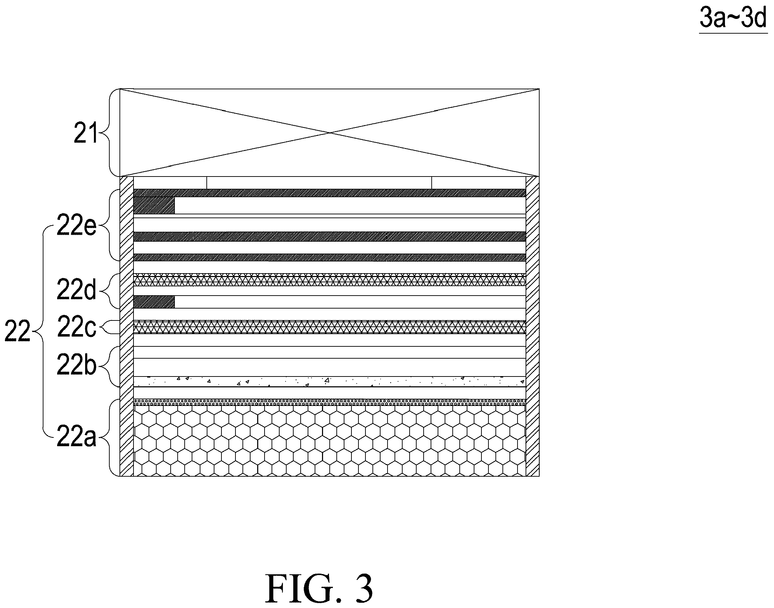

Please refer to FIG. 3. Each of the filtration and purification devices 3a, 3b, 3c, 3d includes a flow-guiding component 21 and a filtration and purification module 22. The flow-guiding component 21 guides the polluted gas to pass through the filtration and purification module 22 for filtration and purification. The flow-guiding component 21 may be at least one selected from an electric fan, a cleaner, an air conditioner, and a fresh air purifier. Moreover, the volume of each of the filtration and purification devices 3a, 3b, 3c, 3d at the areas A, B, C, D in the indoor space is in a range between 16.5 and 247.5 m³, the number of the filtration and purification devices 3a, 3b, 3c, 3d is between 2 and 75, and the clean air delivery rate (CADR) of the flow-guiding component 21 of each of the filtration and purification devices 3a, 3b, 3c, 3d is in a range between 200 and 1600, therefore the polluted gas can be cleaned by each of the filtration and purification devices 3a, 3b, 3c, 3d in one minute to achieve a concentration of PM2.5 which is less than 10 µg/m³, a concentration of carbon dioxide which is less than 1000 ppm, a concentration of total volatile organic compounds (TVOC) which is less than 0.56 ppm, a concentration of formaldehyde which is less than 0.08 ppm, a colony-forming unit of bacteria which is less than 1500 CFU/m³, a colony-forming unit of fungi which is less than 1000 CFU/m³, a concentration of sulfur dioxide which is less than 0.075 ppm, a concentration of nitrogen dioxide which is less than 0.1 ppm, a concentration of carbon monoxide which is less than 35 ppm, a concentration of ozone which is less than 0.12 ppm, and a concentration of lead which is less than 0.15 µg/m³.

Moreover, the filtration and purification module 22 may be the combination of various embodiments. In one embodiment, the filtration and purification module 22 may be a high-efficiency particulate air (HEPA) filter 22a. The chemical smog, bacteria, dusts, particles, and pollens contained in the polluted gas are absorbed by the high-efficiency particulate air filter 22a, thereby the polluted gas introduced into the filtration and purification module 22 is filtered and purified. In some embodiments, a cleansing factor layer having chlorine dioxide is coated on the high-efficiency particulate air filter 22a for suppressing viruses, bacteria, fungus, influenza A virus, influenza B virus, Enterovirus, and Norovirus in the polluted gas introduced into the filtration and purification module 22. Accordingly, the suppressing rate may exceed 99%, thereby allowing the reduction of the cross infections of the microorganisms as mentioned above. In some other embodiments, a herbal protection coating layer including the extracts of Rhus chinensis Mill (can be Rhus chinensis Mill from Japan) and the extracts of Ginkgo biloba may be coated on the high-efficiency particulate air filter 22a to form a herbal protection anti-allergy filter which can efficiently perform anti-allergy function and destroy cell surface proteins of influenza viruses (e.g., influenza virus subtype H1N1) passing through the herbal protection anti-allergy filter. Alternatively, in some other embodiments, a layer of silver ions may be coated on the high-efficiency particulate air filter 22a for suppressing viruses, bacteria, and fungus in the polluted gas introduced by the filtration and purification module 22.

In another embodiment, the filtration and purification module 22 may be a combination of the high-efficiency particulate air filter 22a and a photocatalyst unit 22b. Therefore, the polluted gas in the indoor space is introduced into the filtration and purification module 22, hazardous matters in the polluted gas are degraded and sterilized, such that the polluted gas is filtered and purified by the filtration and purification module 22 through the photocatalyst unit 22b by converting the light energy into chemical energy.

In another embodiment, the filtration and purification module 22 may be a combination of the high-efficiency particulate air filter 22a and a photo plasma unit 22c. The photo plasma unit 22c includes a nanometer light tube. Through illuminating the polluted gas introduced from the filtration and purification module 22 with the light irradiated from the nanometer light tube, the volatile organic gases contained in the polluted gas can be degraded and purified. When the polluted gas is illuminated by the light irradiated from the nanometer light tube, the oxygen molecules and water molecules in the polluted gas are degraded into high oxidative photo plasma, forming a plasma stream, which is capable destroying organic molecules. Accordingly, volatile organic compounds (VOC) such as formaldehyde and toluene in the polluted gas can be degraded into water and carbon dioxide. Thus, the polluted gas can be filtered and purified by the filtration and purification module 22.

In another embodiment, the filtration and purification module 22 may be a combination of the high-efficiency particulate air filter 22a and a negative ion unit 22d. Through applying high voltage discharging to the polluted gas introduced into the filtration and purification module 22, the particulates with positive charges in the polluted gas are adhered to the dust-collecting plate with negative charges for filtering and purifying the polluted gas.

In another embodiment, the filtration and purification module 22 may be a combination of the high-efficiency particulate air filter 22a and a plasma ion unit 22e. The plasma ion unit 213e generates a high-voltage plasma. Therefore, the viruses and the bacteria in the polluted gas introduced into the filtration and purification module 22 are degraded by the high-voltage plasma. Accordingly, the oxygen molecules and the water molecules in the polluted gas are ionized to form cations (H⁺) and anions (O²⁻) through the high-voltage plasma. The substances attached with water molecules around the ions are attached on the surfaces of viruses and bacteria, converting the water molecules into oxidative oxygen ions (hydroxyl ions, OH⁻ ions), and the oxidative oxygen ions take away the hydrogen ions of the proteins on the surfaces of the viruses and the bacteria to degrade the microorganisms as mentioned above. Accordingly, the polluted gas is filtered and purified by the filtration and purification module 22.

In one embodiment, the filtration and purification module 22 may only include the high-efficiency particulate air filter 22a. Alternatively, in another embodiment, the filtration and purification module 22 may be a combination of the high-efficiency particulate air filter 22a and any one of the photocatalyst unit 22b, the photo plasma unit 22c, the negative ion unit 22d, and the plasma ion unit 22e. In one embodiment, the filtration and purification module 22 may be a combination of the high-efficiency particulate air filter 22a and any two of the photocatalyst unit 22b, the photo plasma unit 22c, the negative ion unit 22d, and the plasma ion unit 22e. In one embodiment, the filtration and purification module 22 may be a combination of the high-efficiency particulate air filter 22a and any three of the photocatalyst unit 22b, the photo plasma unit 22c, the negative ion unit 22d, and the plasma ion unit 22e. In one embodiment, the filtration and purification module 22 may be a combination of the high-efficiency particulate air filter 22a and all of the photocatalyst unit 22b, the photo plasma unit 22c, the negative ion unit 22d, and the plasma ion unit 22e.

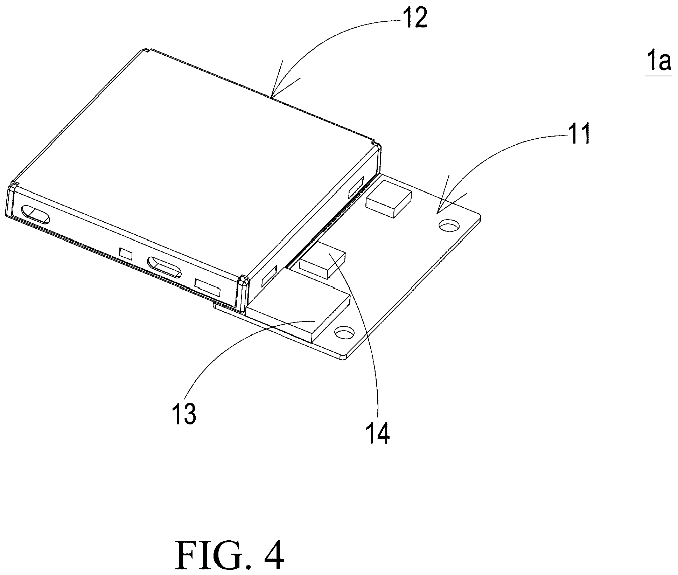

As shown in FIG. 4, the gas detection devices la, 1b, 1c, 1d detect and transmit the data of the polluted gas. In the following embodiment, the structure of the gas detection device 1a is described, further, in one aspect of the present disclosure, the structures of the gas detection devices 1b, 1c, 1d are the same as the gas detection device 1a. The gas detection device 1a includes a control circuit board 11, a gas detection main body 12, a microprocessor 13, and a communication device 14. Wherein the gas detection main body 12, the microprocessor 13, and the communication device 14 are integrally packaged with the control circuit board 11 and electrically connected to each other. The microprocessor 13 and the communication device 14 are disposed on the control circuit board 11. The microprocessor 13 controls the driving signal of the gas detection main body 12 to enable the gas detection main body 12, receives the information of the polluted gas detected by the gas detection device 1a for computation, communicates outwardly through the communication device 14, and converts the information into a gas detection data for storage. The communication device 14 receives the gas detection data outputted from the microprocessor 13 and transmits the gas detection data to a cloud processing device 2b or to an external device (which may be a mobile device 2a). Specifically, in this embodiment, the communication device 14 can be communicationally connected to the cloud processing device 2b to transmit data, and the data transmission may be achieved in accordance with the predetermined areas A, B, C, D in the indoor space. Moreover, in some embodiments, the outwardly communication transmission of the communication devices 14 may be implemented through a bidirectional wired transmission. For example, the wired transmission may be achieved by a USB port, a mini-USB port, and micro-USB port. The outwardly communication transmission of the communication devices 14 may also be implemented through a bidirectional wireless transmission. For example, the wireless transmission may be achieved by a Wi-Fi module, a Bluetooth module, a radiofrequency identification module, and a near field communication module.

The polluted gas may include at least one selected from the group consisting of particulate matters, carbon monoxide (CO), carbon dioxide (CO₂), ozone (O₃), sulfur dioxide (SO₂), nitrogen dioxide (NO₂), lead (Pb), total volatile organic compounds (TVOC), formaldehyde (HCHO), bacteria, fungi, and viruses.

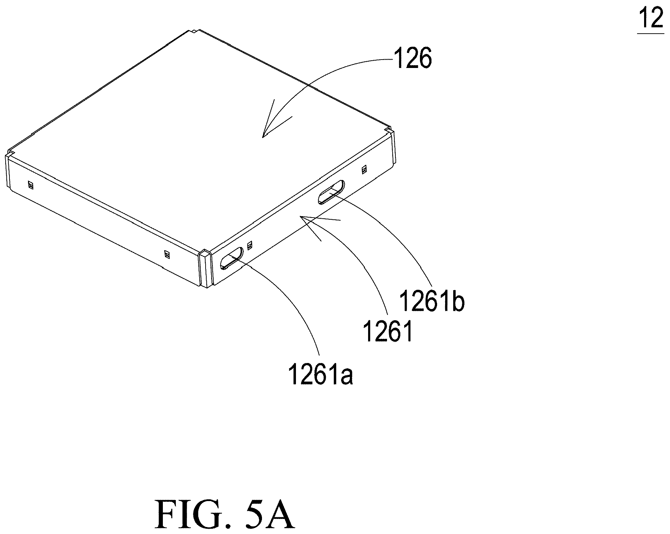

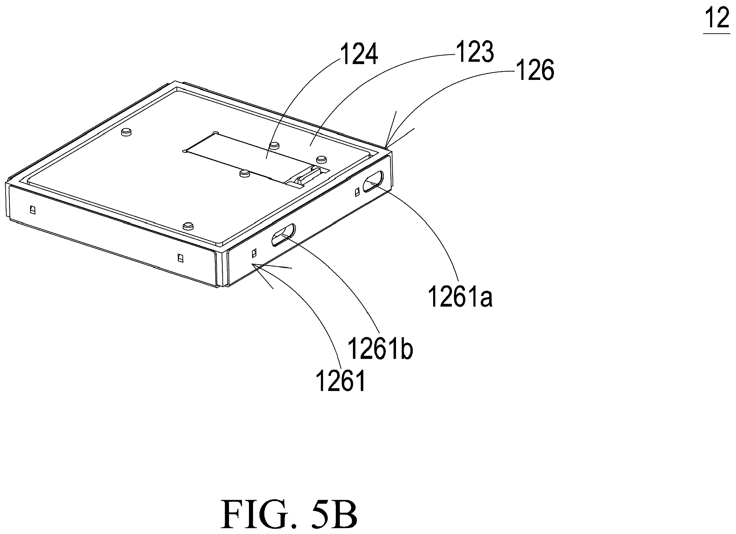

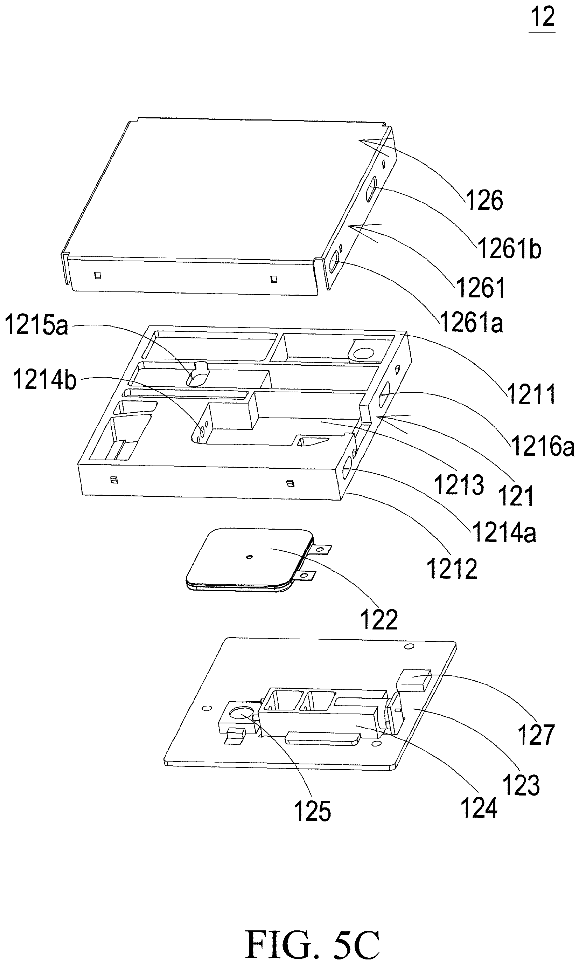

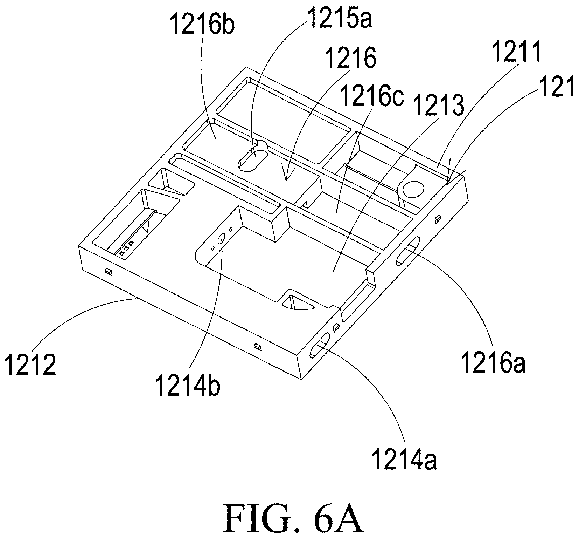

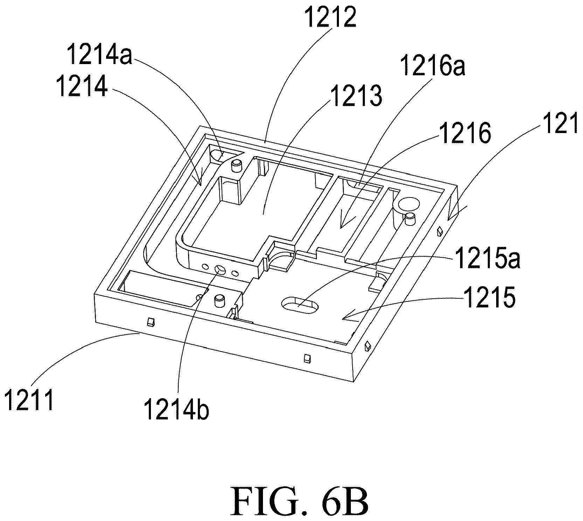

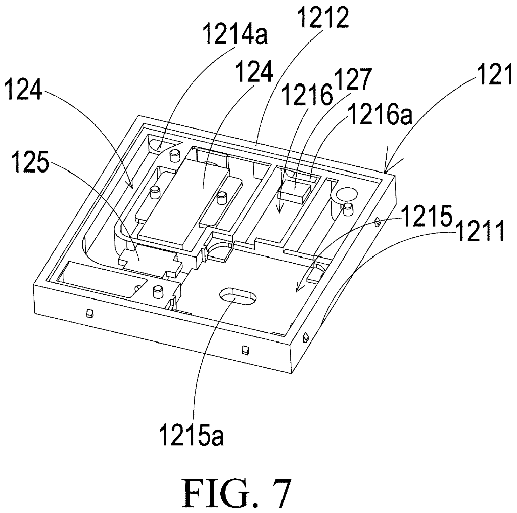

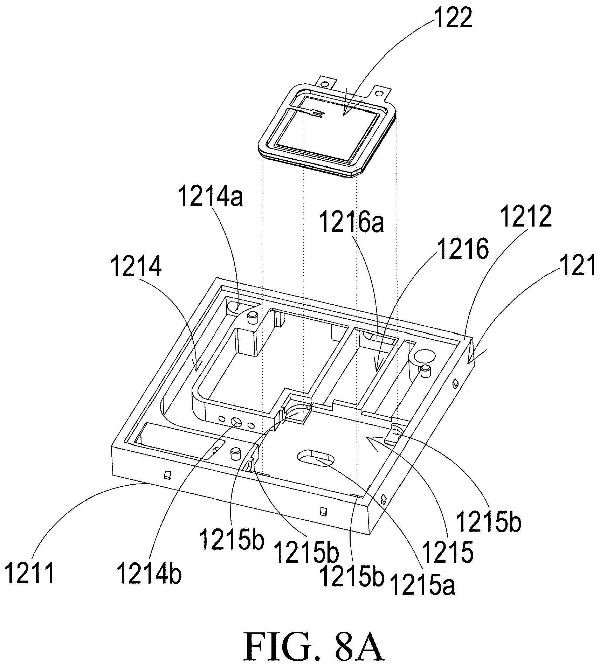

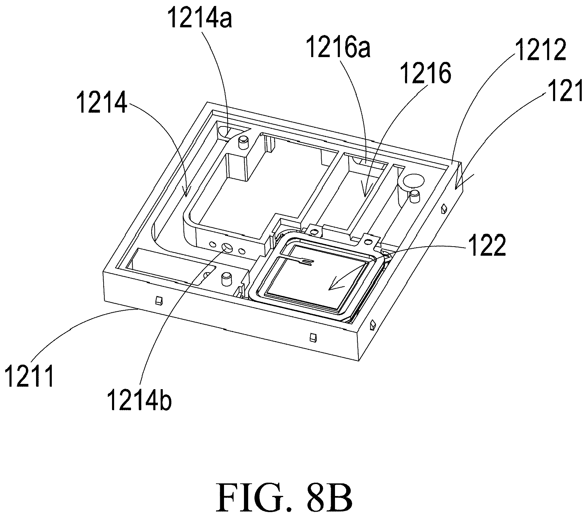

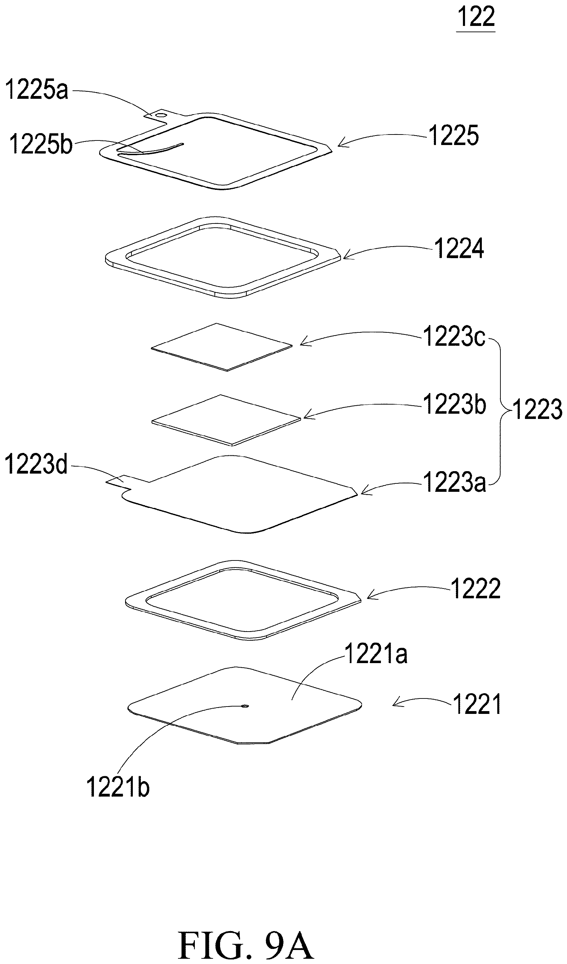

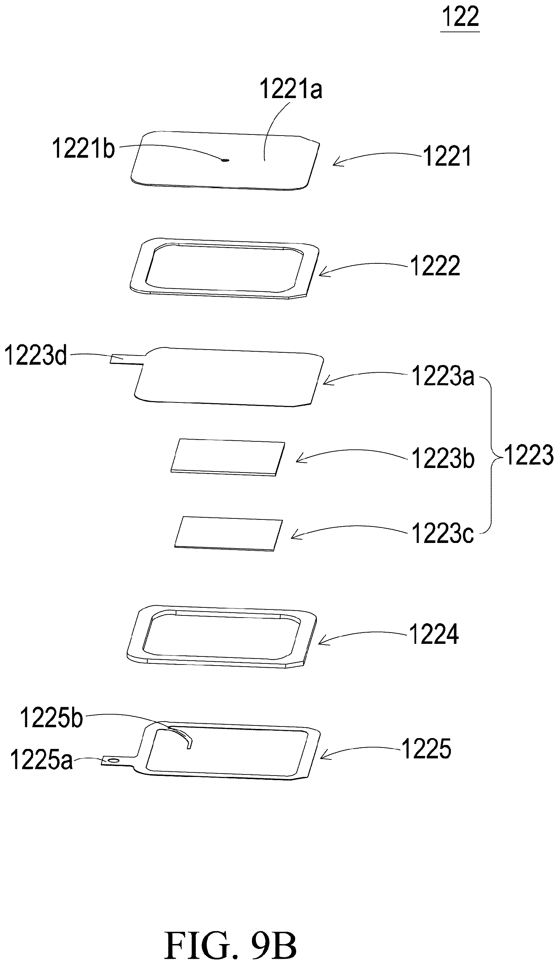

Please refer to FIG. 5A to FIG. 10A for further illustration. The gas detection main body 12 includes a base 121, a piezoelectric actuator 122, a driving circuit board 123, a laser component 124, a particulate sensor 125, an outer cap 126, and a gas sensor 127. The base 121 has a first surface 1211, a second surface 1212, a laser configuration region 1213, a gas inlet groove 1214, a gas-guiding component loading region 1215, and a gas outlet groove 1216. Wherein the first surface 1211 and the second surface 1212 are opposite to each other. The laser configuration region 1213 is hollowed out from the first surface 1211 to the second surface 1212 for accommodating the laser component 124. The outer cap 126 covers the base 121 and has a side plate 1261. The side plate 1261 has a gas inlet opening 1261a and a gas outlet opening 1261b. The gas inlet groove 1214 is recessed from the second surface 1212 and located adjacent to the laser configuration region 1213. The gas inlet groove 1214 has a gas inlet through hole 1214a and two lateral walls. The gas inlet through hole 1214a penetrates inside and outside of the base 121 and corresponds to the gas inlet opening 1261a of the outer cap 126. Two light permissive windows 1214b penetrate the two lateral walls of the gas inlet groove 1214 and are in communication with the laser configuration region 1213. Therefore, the first surface 1211 of the base 121 is covered by the outer cap 126, and the second surface 1212 of the base 121 is covered by the driving circuit board 123, therefore, a gas inlet path with the gas inlet groove 1214 can be defined result from the aforementioned structure.

The gas-guiding component loading region 1215 is recessed from the second surface 1212 and in communication with the gas inlet groove 1214. A gas flowing hole 1215a penetrates a bottom surface of the gas-guiding component loading region 1215. Each of four corners of the gas-guiding component loading region 1215 has a positioning bump 1215b. The gas outlet groove 1216 has a gas outlet through hole 1216a, and the gas outlet through hole 1216a is corresponding to the gas outlet opening 1261b of the outer cap 126. The gas outlet groove 1216 includes a first region 1216b and a second region 1216c. The first region 1216b is recessed from a portion of the first surface 1211 corresponding to a vertical projection region of the gas-guiding component loading region 1215. The second region 1216c is at a portion extending from a portion not corresponding to the vertical projection region of the gas-guiding component loading region 1215, and the second region 1216c is hollowed out from the first surface 1211 to the second surface 1212 in a region where the first surface 1211 is not aligned with the gas-guiding component loading region 1215. The first region 1216b is connected to the second region 1216c to form a stepped structure. Moreover, the first region 1216b of the gas outlet groove 1216 is in communication with the gas flowing hole 1215a of the gas-guiding component loading region 1215, and the second region 1216c of the gas outlet groove 1216 is in communication with the gas outlet through hole 1216a. Therefore, when the first surface 1211 of the base 121 is covered by the outer cap 126 and the second surface 1212 of the base 121 is covered by the driving circuit board 123, the gas outlet groove 1216 and the driving circuit board 123 together define a gas outlet path.

Furthermore, the laser component 124 and the particulate sensor 125 are disposed on the driving circuit board 123 and located in the base 121, wherein the laser component 124 and the particulate sensor 125 are electrically connected to the driving circuit board 123. One should notice that to clearly explain the positions of the laser component 124, the particulate sensor 125, and the base 121, the driving circuit board 123 is not illustrated. In the embodiment of the present disclosure, the laser component 124 is located at the laser configuration region 1213 of the base 121. The particulate sensor 125 is located at the gas inlet groove 1214 of the base 121 and aligned with the laser component 124. Moreover, the laser component 124 corresponds to the light permissive windows 1214b, allowing the light beam emitted by the laser component 124 to pass therethrough into the gas inlet groove 1214. The path of the light beam emitted by the laser component 124 passes through the light permissive windows 1214b and is orthogonal to the gas inlet groove 1214. The light beam emitted by the laser component 124 pass through into the gas inlet groove 1214 by the light permissive windows 1214b, and the particulate matters in the gas inlet groove 1214 is illuminated by the light beam. When the light beam encounters the particulate matters, the light beam will be scattered to generate light spots. Hence, the particulate sensor 125 receives and calculates the light spots generated by the scattering to obtain the detection data of the gas (particulates information). Furthermore, the gas sensor 127 is disposed on the driving circuit board 123, and is located at the gas outlet groove 1216 for detecting the polluted gas introduced into the gas outlet groove 1216, wherein the gas sensor 127 is electrically connected to the driving circuit board 123. In one embodiment of the present disclosure, the gas sensor 127 includes at least one selected from the group consisting of a volatile organic compound detector capable of detecting gas information of carbon dioxide (CO₂) or total volatile organic compounds (TVOC), a formaldehyde sensor capable of detecting gas information of formaldehyde (HCHO) gas, a bacterial sensor capable of detecting information of bacteria or fungi, and a virus sensor capable of detecting information of viruses.

Moreover, the piezoelectric actuator 122 is located at the gas-guiding component loading region 1215 with square-shaped of the base 121, wherein the gas-guiding component loading region 1215 is interconnected with the gas inlet groove 1214. When the piezoelectric actuator 122 operates, the gas in the gas inlet groove 1214 is driven into the piezoelectric actuator 122, thereby the gas would pass through the gas flowing hole 1215a of the gas-guiding component loading region 1215, and enter into the gas outlet groove 1216. Moreover, the driving circuit board 123 covers the second surface 1212 of the base 121. The laser component 124 and particulate sensor 125 are disposed on the driving circuit board 123, also the laser component 124 and particulate sensor 125 are electrically connected to the driving circuit board 123. As the outer cap 126 covers the base 121, the gas inlet opening 1216a is corresponding to the gas inlet through hole 1214a of the base 121, and the gas outlet opening 1216b is corresponding to the gas outlet through hole 1216a of the base 121.

Furthermore, the piezoelectric actuator 122 includes a nozzle plate 1221, a chamber frame 1222, an actuation body 1223, an insulation frame 1224, and a conductive frame 1225. The nozzle plate 1221 is made by a flexible material and has a suspension sheet 1221a and a hollow hole 1221b. The suspension sheet 1221a is a flexible sheet, which can bend and vibrate. The shape and the size of the suspension sheet 1221a approximately correspond to those of the inner edge of the gas-guiding component loading region 1215. The hollow hole 1221b penetrates through the center portion of the suspension sheet 1221a for the gas flowing therethrough. In one preferred embodiment of the present invention, the shape of the suspension sheet 1221a can be selected from square, circle, ellipse, triangle, and polygon.

Furthermore, the chamber frame 1222 is stacked on the nozzle plate 1221, and the shape of the chamber frame 1222 is corresponding to the shape of the nozzle plate 1221. The actuation body 1223 is stacked on the chamber frame 1222. A resonance chamber 1226 is defined through the stack structure of the actuation body 1223, the nozzle plate 1221, and the suspension sheet 1221a. The insulation frame 1224 is stacked on the actuation body 1223. The appearance of the insulation frame 1224 is similar to the appearance of the nozzle plate 1221. The conductive frame 1225 is stacked on the insulation frame 1224. The appearance of the conductive frame 1225 is similar to the appearance of the insulation frame 1224. The conductive frame 1225 has a conductive frame pin 1225a and a conductive electrode 1225b. The conductive frame pin 1225a extends outwardly from the outer edge of the conductive frame 1225, and the conductive electrode 1225b extends inwardly from the inner edge of the conductive frame 1225.

Moreover, the actuation body 1223 further includes a piezoelectric carrier plate 1223a, an adjusting resonance plate 1223b, and a piezoelectric plate 1223c. Wherein the piezoelectric carrier plate 1223a is stacked on the chamber frame 1222; the adjusting resonance plate 1223b is stacked on the piezoelectric carrier plate 1223a; the piezoelectric plate 1223c is stacked on the adjusting resonance plate 1223b. The adjusting resonance plate 1223b and the piezoelectric plate 1223c are accommodated in the insulation frame 1224. The conductive electrode 1225b of the conductive frame 1225 is electrically connected to the piezoelectric plate 1223c. In one embodiment, the piezoelectric carrier plate 1223a and the adjusting resonance plate 1223b are both made of the same conductive material or different conductive materials. The piezoelectric carrier plate 1223a has a piezoelectric pin 1223d. The piezoelectric pin 1223d and the conductive frame pin 1225a are electrical connection with a driving circuit (not shown) of the driving circuit board 123 to receive a driving signal (which may be a driving frequency and a driving voltage). The piezoelectric pin 1223d, the piezoelectric carrier plate 1223a, the adjusting resonance plate 1223b, the piezoelectric plate 1223c, the conductive electrode 1225b, the conductive frame 1225, and the conductive frame pin 1225a may together form an electrical circuit for transmitting the driving signal, and the insulation frame 1224 is provided for electrically isolating the conductive frame 1225 from the actuation body 1223 for avoiding short circuit, thereby the driving signal can be transmitted to the piezoelectric plate 1223c. When the piezoelectric plate 1223c receives the driving signal, the piezoelectric plate 1223c deforms owing to the piezoelectric effect, and thus the piezoelectric carrier plate 1223a and the adjusting resonance plate 1223b are driven to perform reciprocating vibration correspondingly.

Moreover, the adjusting resonance plate 1223b is disposed between the piezoelectric plate 1233c and the piezoelectric carrier plate 1223a as a cushion element so as to adjust the vibration frequency of the piezoelectric carrier plate 1223a. Generally, the thickness of the adjusting resonance plate 1223b is greater than the thickness of the piezoelectric carrier plate 1223a. The thickness of the adjusting resonance plate 1223b may be changed to adjust the vibration frequency of the actuation body 1223. The nozzle plate 1221, the chamber frame 1222, the actuation body 1223, the insulation frame 1224, and the conductive frame 1225 are sequentially stacked and assembled, making the piezoelectric actuator 122 be placed and positioned in the gas-guiding component loading region 1215, thus, a clearance 1221c is defined between the suspension sheet 1221a and the inner edge of the gas-guiding component loading region 1215 for the gas to pass therethrough.

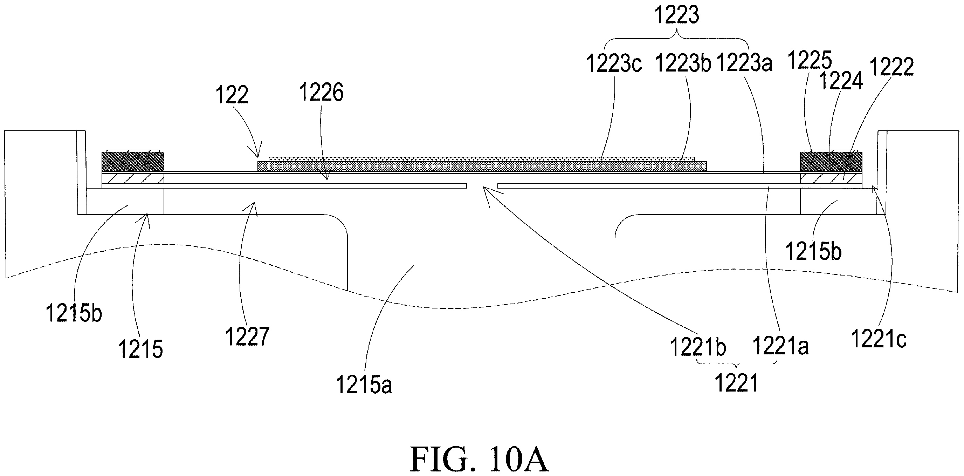

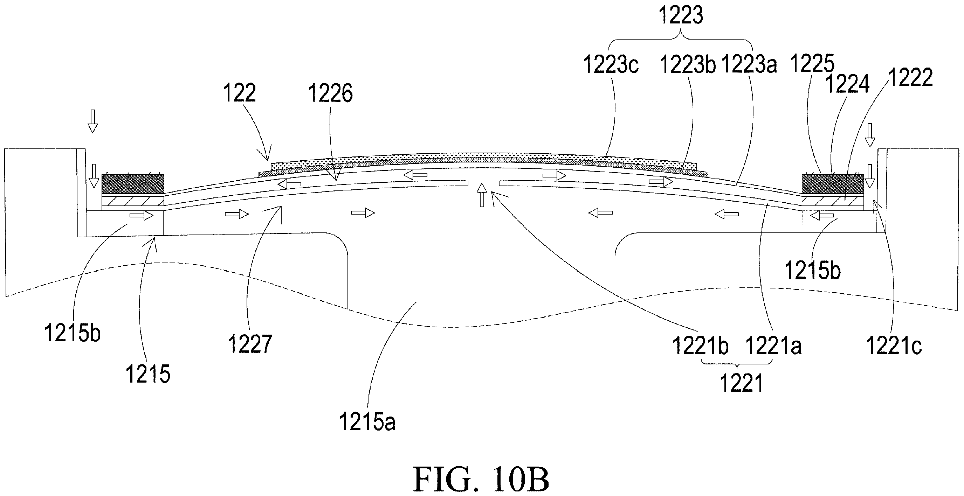

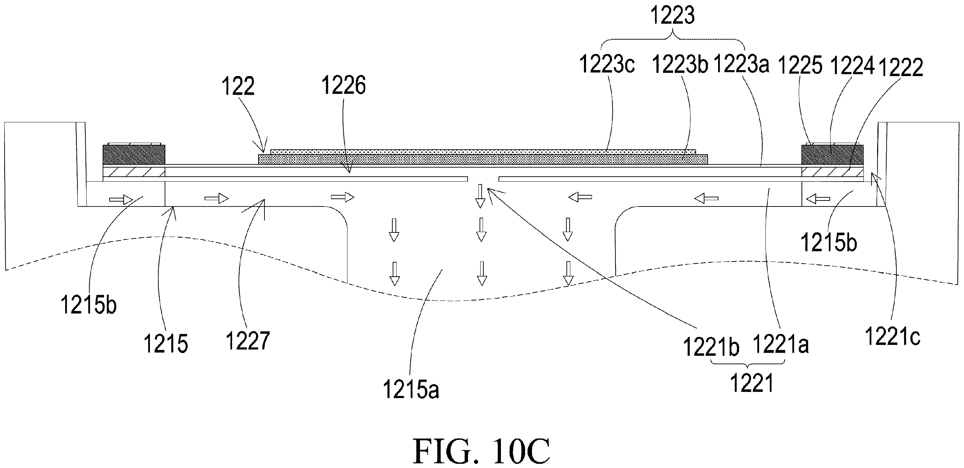

Please refer to the FIG. 10A, FIG. 10B, and FIG. 10C, a gas flow chamber 1227 is formed between a bottom of the nozzle plate 1221 and the bottom surface of the gas-guiding component loading region 1215. The gas flow chamber 1227 connect with the resonance chamber 1226 formed between the actuation body 1223, the nozzle plate 1221, and the suspension sheet 1221a through the hollow hole 1221b of the nozzle plate 1221. In one aspect of the present invention, the resonance chamber 1226 and the suspension sheet 1221a can generate the Helmholtz resonance effect to improve the transmission efficiency of the gas through the vibration frequencies of the gas in the resonance chamber 1226 and suspension sheet 1221a are approaching the same. When the piezoelectric plate 1223c moves in a direction away from the bottom surface of the gas-guiding component loading region 1215, the piezoelectric plate 1223c drives the suspension sheet 3221a of the nozzle plate 1221 to move in the direction away from the bottom surface of the gas-guiding component loading region 1215 correspondingly. Hence, the volume of the gas flow chamber 1227 expands dramatically, so that the internal pressure of the gas flow chamber 1227 decreases and creates a negative pressure, drawing the gas outside the piezoelectric actuator 122 to flow into the piezoelectric actuator 122 through the clearance 1221c and enter into the resonance chamber 1226 through the hollow hole 1221b, thereby increasing the gas pressure of the resonance chamber 1226 and thus generating a pressure gradient. When the piezoelectric plate 1223c drives the suspension sheet 1221a of the nozzle plate 1221 to move toward the bottom surface of the gas-guiding component loading region 1215, the gas inside the resonance chamber 1226 is pushed to flow out quickly through the hollow hole 1221b to further push the gas inside the gas flow chamber 1227, thereby the converged gas can be quickly and massively ejected out of the gas flow chamber 1227 and introduced into the gas flowing hole 1215a of the gas-guiding loading region 1215 in a state closing to an ideal gas state under the Benulli's law.

Therefore, through repeating the steps as shown in FIG. 10B and FIG. 10C, the piezoelectric plate 1223c can bend and vibrate reciprocatingly. Furthermore, after the gas is discharged out of the resonance chamber 1226, the internal pressure of the resonance chamber 1226 is lower than the equilibrium pressure due to the inertia, as a result, the pressure difference guides the gas outside the resonance chamber 1226 into the resonance chamber 1226 again. Thus, the resonance chamber 1226 and the piezoelectric plate 1223c can generate the Helmholtz resonance effect to achieve effective, high-speed, and large-volume gas transmission of the gas when the vibration frequencies of the gas in the resonance chamber 1226 and piezoelectric plate 1223c are approaching the same.

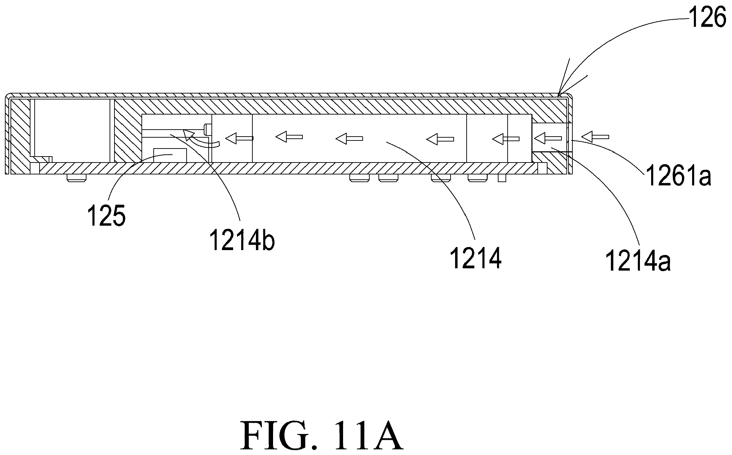

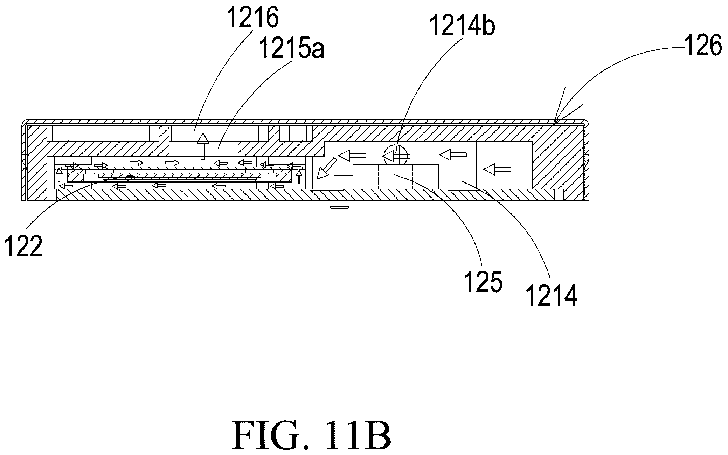

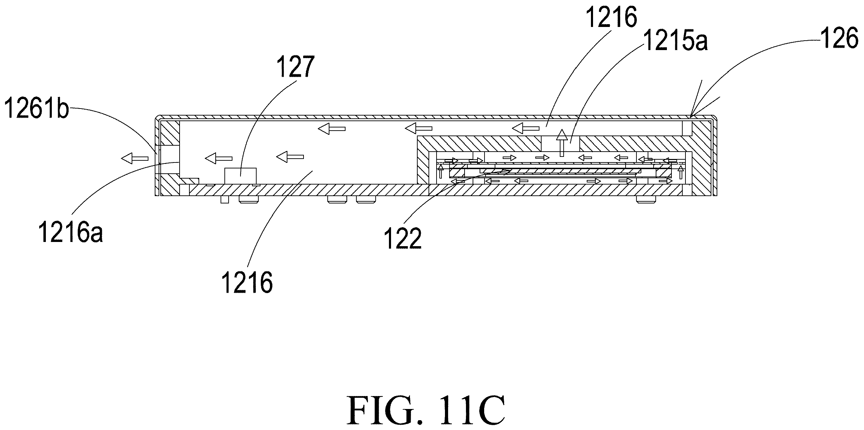

Moreover, as shown in FIG. 11A, FIG. 11B and FIG. 11C, the gas enters into the gas inlet opening 1214a from the outer cap 126, which flow into the gas inlet groove 1214 of the base 121 through the gas inlet opening 1214a for reaching the position of the particulate sensor 125. Furthermore, the piezoelectric actuator 122 continuously drives the gas into the gas inlet path to facilitate the gas inside the detection main body 12 stably and quickly pass through the particulate sensor 125. Next, the light beam emitted by the laser component 124 passes through the light permissive windows 1214b and enters into the gas inlet groove 1214. The gas in the gas inlet groove 1214 that passes through the particulate sensor 125 is illuminated by the light beam. When the light beam encounters the particulate matters in the gas, the light beam will be scattered to generate light spots. The particulate sensor 125 receives and calculates the light spots generated by the scattering, to obtain the information such as the particle size and the concentration of the particulate matters in the gas. Moreover, the gas passing through the particulate sensor 125 is continuously introduced into the gas flowing hole 1215a of the gas-guiding component loading region 1215 by the driving of the piezoelectric actuator 122 and enters into the gas outlet groove 1216. Finally, after the gas enters into the gas outlet groove 1216, since the piezoelectric actuator 122 continuously delivers the gas into gas outlet groove 1216, therefore the gas is pushed continuously, which discharged out of the gas detection main body 12 eventually through the gas outlet through hole 1216a and the gas outlet opening 1261b.

As noted above, in one or some embodiments of the present disclosure, a plurality of gas detection devices and a plurality of filtration and purification devices corresponding to the gas detection devices are disposed in the indoor space. The gas detection devices detect the data of the polluted gas in the indoor space and transmits the data to the connection device. The connection device receives and compares the data of the polluted gas detected by the gas detection devices to perform intelligent computation to locate a location in the indoor space containing the polluted gas. Moreover, the connection device intelligently and selectively transmits a first control command to a filtration and purification device at the location of the indoor space containing the polluted gas to enable the filtration and purification device to filter the polluted gas, allowing the polluted gas in the indoor space to become a clean, safe, and breathable gas.

## Claims

1. A method for detecting and filtering indoor polluted gas adapted to figure out a polluted gas in an indoor space for filtration, **characterized in that** the method comprises:
providing a plurality of gas detection devices (1a, 1b, 1c, 1d) to detect the polluted gas, wherein each of the gas detection devices (1a, 1b, 1c 1d) detect a data of the polluted gas in the indoor space for outputting the data;
providing a plurality of filtration and purification devices (3a, 3b, 3c, 3d) to filter the polluted gas, wherein each of the filtration and the purification devices receives (3a, 3b, 3c, 3d) a first control command through a wireless communication or a wired communication to enable filtration of the polluted gas; and
providing a connection device (2) to perform intelligent computation, wherein the connection device (2) receives and compares the data of the polluted gas detected by the gas detection devices (1a, 1b, 1c, 1d) to perform intelligent computation for finding out a location in the indoor space containing the polluted gas and to intelligently and selectively transmit the first control command to a filtration and purification device (3a, 3b, 3c, 3d) at the location of the indoor space containing the polluted gas;
wherein the filtration and purification device (3a, 3b, 3c, 3d) at the location of the indoor space containing the polluted gas is enabled to filter the polluted gas and retain the polluted gas from spreading outwardly, allowing the polluted gas in the indoor space to become a clean, safe, and breathable gas.

2. The method for detecting and filtering indoor polluted gas according to claim 1, wherein the polluted gas comprises at least one selected from the group consisting of particulate matters, carbon monoxide, carbon dioxide, ozone, sulfur dioxide, nitrogen dioxide, lead, total volatile organic compounds, formaldehyde, bacteria, fungi, and viruses.

3. The method for detecting and filtering indoor polluted gas according to claim 1, wherein the connection device (2) intelligently and selectively transmits the first control command to and enable the filtration and purification device (3a, 3b, 3c, 3d) at the location containing the polluted gas; wherein the connection device (2) intelligently and selectively transmits a second control command to and enable rest of the filtration and purification devices (3a, 3b, 3c, 3d) to accelerate the filtration of the polluted gas, allowing the polluted gas in the indoor space to become a clean, safe, and breathable gas.

4. The method for detecting and filtering indoor polluted gas according to claim 1, wherein each of the gas detection devices (1a, 1b, 1c, 1d) is disposed in the indoor space and is stationary or movable.

5. The method for detecting and filtering indoor polluted gas according to claim 1, wherein the connection device (2) receives and compares the data of the polluted gas in the indoor space detected by at least three of the gas detection devices (1a, 1b, 1c, 1d), the connection device (2) intelligently computes the data having a highest value among the data to determine and selectively figure out the location of the indoor space containing the polluted gas.

6. The method for detecting and filtering indoor polluted gas according to claim 1, wherein each of the gas detection devices (1a, 1b, 1c, 1d) comprises a control circuit board (11), a gas detection main body (12), a microprocessor (13), and a communication device (14); the gas detection main body (12), the microprocessor (13), and the communication device (14) are integrally packaged with the control circuit board (11) and electrically connected to each other; the microprocessor (13) controls the detection of the gas detection main body (12), the gas detection main body (12) detects the polluted gas to output a detection signal, and the microprocessor (14) receives the detection signal to perform computation to output the data of the polluted gas to the communication device (14) for wirelessly transmitting outwardly.

7. The method for detecting and filtering indoor polluted gas according to claim 6, wherein the gas detection main body (12) comprises:
a base (121), having:
a first surface (1211);
a second surface (1212) opposite to the first surface (1211);
a laser configuration region (1213) hollowed out from the first surface (1211) to the second surface (1212);
a gas inlet groove (1214) recessed from the second surface (1212) and located adjacent to the laser configuration region (1213), wherein the gas inlet groove (1214) has a gas inlet through hole (1214a) and two lateral walls; two light permissive windows (1214b) penetrate on the two lateral walls of the gas inlet groove (1214) and in communication with the laser configuration region (1213);
a gas-guiding component loading region (1215) recessed from the second surface (1212) and in communication with the gas inlet groove (1214), wherein a gas flowing hole (1215a) penetrates a bottom surface of the gas-guiding component loading region (1215); and
a gas outlet groove (1216) includes a first region (1216b), corresponding to the gas-guiding component loading region (1215), recessed from a portion of the first surface (1211) corresponding to the bottom surface of the gas-guiding component loading region (1215); and a second region (1216c), not corresponding to the gas-guiding component loading region (1215), hollowed out from the first surface (1211) to the second surface (1212) in a region where the first surface (1211) is not aligned with the gas-guiding component loading region (1215), wherein the gas outlet groove (1216) is in communication with the gas flowing hole (1215a) and has a gas outlet through hole (1215a);
a piezoelectric actuator (122) received in the gas-guiding component loading region (1215);
a driving circuit board (123) attached to the second surface (1212) of the base (121);
a laser component (124) disposed on the driving circuit board (123) and electrically connected to the driving circuit board (123), wherein the laser component (124) is received in the laser configuration region (1213), and a path of a light beam emitted by the laser component (124) passes through the light permissive windows (1214b) and is orthogonal to the gas inlet groove (1214);
a particulate sensor (125) disposed on the driving circuit board (123) and electrically connected to the driving circuit board (123), wherein the particulate sensor (125) is received in a portion of the gas inlet groove (1214) where the path of the light beam emitted by the laser component (124) is orthogonal thereto, therefore the particulate sensor (125) detects particulates in the polluted gas passing through the gas inlet groove (1214) which is illuminated by the light beam of the laser component (124);
a gas sensor (127) disposed on and electrically connected to the driving circuit board (123), wherein the gas sensor (127) is received in the gas outlet groove (1216), so that the gas sensor (127) detects the polluted gas introduced into the gas outlet groove (1216); and
an outer cap (126) covering the base and having a side plate (1261), and the side plate (1261) has a gas inlet opening (1261a) and a gas outlet opening (1261b), the gas inlet opening (1261a) is corresponding to the gas inlet through hole (1214a) of the base (121), and the gas outlet opening (1261b) is corresponding to the gas outlet through hole (1216a) of the base (121);
wherein the outer cap (126) is covered on the base (121), and the driving circuit board (123) is attached to the second surface (1212) of the base (121), so that the gas inlet groove (1214) defines a gas inlet path and the gas outlet groove (1216) defines a gas outlet path, thereby facilitating the piezoelectric actuator (122) to introduce the polluted gas outside the gas inlet through hole (1214a) of the base (121) into the gas inlet path defined by the gas inlet groove (1214) from the gas inlet opening (1216a); the polluted gas passes through the particulate sensor (125) to detect a particle concentration of the particulates in the polluted gas; and the polluted gas is discharged into the gas outlet path defined by the gas outlet groove (1216) from the gas flowing hole (1215a), detected by the gas sensor (127), and is discharged out of the gas detection main body (12) from the gas outlet through hole (1216a) and the gas outlet opening (1216b) of the base (121).

8. The method for detecting and filtering indoor polluted gas according to claim 7, wherein the particulate sensor (125) is capable of detecting particulate matters.

9. The method for detecting and filtering indoor polluted gas according to claim 7, wherein the gas sensor (127) comprises a volatile organic compound detector capable of detecting carbon dioxide or total volatile organic compounds.

10. The method for detecting and filtering indoor polluted gas according to claim 7, wherein the gas sensor (127) comprises at least one selected from the group consisting of a formaldehyde sensor, a bacterial sensor, and a virus sensor; the formaldehyde sensor is capable of detecting formaldehyde gas; the bacterial sensor is capable of detecting bacteria or fungi; the virus sensor is capable of detecting viruses.

11. The method for detecting and filtering indoor polluted gas according to claim 1, wherein the connection device (2) is selected from the group consisting of a mobile device (2a) and a cloud processing device (2b), wherein the wireless communication is achieved by one of a Wi-Fi module, a Bluetooth module, a radiofrequency identification module, and a near field communication module.

12. The method for detecting and filtering indoor polluted gas according to claim 1, wherein each of the filtration and purification devices (3a, 3b, 3c, 3d) comprises a flow-guiding component (21) and a filtration and purification module (22); the flow-guiding component (21) guides the polluted gas to pass through the filtration and purification module (22) for filtration and purification.

13. The method for detecting and filtering indoor polluted gas according to claim 12, wherein the flow-guiding component (21) is at least one selected from the group consisting of an electric fan, a cleaner, an air conditioner, and a fresh air purifier.

14. The method for detecting and filtering indoor polluted gas according to claim 12, wherein the volume of each of the filtration and purification devices (3a, 3b, 3c, 3d) in the indoor space is in a range between 16.5 and 247.5 m³, the number of the filtration and purification devices (3a, 3b, 3c, 3d) is in a range between 2 and 75, and the clean air delivery rate of the flow-guiding component (21) of each of the filtration and purification devices (3a, 3b, 3c, 3d) is in a range between 200 and 1600, so that the polluted gas is cleaned by each of the filtration and purification devices (3a, 3b, 3c, 3d) in one minute to have a concentration of PM2.5 which is less than 10 µg/m³, a concentration of carbon dioxide which is less than 1000 ppm, a concentration of total volatile organic compounds which is less than 0.56 ppm, a concentration of formaldehyde which is less than 0.08 ppm, a colony-forming unit of bacteria which is less than 1500 CFU/m³, a colony-forming unit of fungi which is less than 1000 CFU/m³, a concentration of sulfur dioxide which is less than 0.075 ppm, a concentration of nitrogen dioxide which is less than 0.1 ppm, a concentration of carbon monoxide which is less than 35 ppm, a concentration of ozone which is less than 0.12 ppm, and a concentration of lead which is less than 0.15 µg/m³.

15. The method for detecting and filtering indoor polluted gas according to claim 12, wherein the filtration and purification module (22) comprises a high-efficiency particulate air (HEPA) filter (22a).
